# EUROPEAN PATENT APPLICATION

(11) **EP 2 255 858 A1**
(43) Date of publication of application: **01.12.2010**
(21) Application number: 10005614.2
(22) Date of filing: 28.05.2010
(51) Int. Cl.: A63J 5/00

(54) **Fragance dispenser**

(30) Priority: 29.05.2009 GB 0909300
(71) Applicant: Flakt Woods Limited, Colchester Essex CO4 5ZD (GB)
(72) Inventor: Mason, Paul, Colchester, Essex CO4 5ZD (GB); Watts, Ian, Colchester, Essex CO4 5ZD (GB)
(74) Representative: JENSEN & SON

(57) **Abstract**

Apparatus for dispensing an air based gaseous medium to a plurality of outlets 1 includes means to add selectively a substance or substances to said air. The apparatus includes a first manifold to which the air is supplied the manifold 5, 8 having connected thereto a plurality of entraining channels 10-19 each having a container 20 containing a substance to be entrained by the air passing therethrough, each entraining channel having a closure valve 21 selectively operable so that the air is able to pass through a selected channel or channels only.

## Description

The present invention relates to dispensing apparatus and particularly, but not exclusively, to apparatus for dispensing area to a plurality of outlets to which predetermined substances has been added to give a desired scent or fragrance.

It is an objective of the entertainment industry such as the cinema and theatre to provide additional means for enhancing the enjoyment or involvement of the audience in a ticket performance by supplying to the audience spells or frequencies appropriate to the scene being viewed. In the past, experiments have been made with filling the whole cinema or theatre with a fragrance dispensed from a central point but these arrangements have not been successful since it has proved impossible to supply and remove the fragrance quickly enough from the large volume so that the smell provided continues to match the scene being viewed.

The present invention seeks to provide a solution to this problem.

According to the present invention there is provided apparatus for dispensing a gaseous medium to a plurality of outlets, the apparatus including means to add selectively a substance or substances to said gaseous medium, the apparatus including a first manifold to which the gaseous medium is supplied the manifold having connected thereto a plurality of entraining channels each containing a substance to be entrained by the gaseous medium passing therethrough, each entraining channel having a closure valve selectively operable so that the gaseous medium is able to pass through a selected channel or channels only.

Preferably, the apparatus includes a bypass channel to enable pure gaseous medium to be supplied downstream of the containers to purge the channels of entrained substances.

In a preferred embodiment, the closure valves are mounted on the manifold so as to be in fluid communication with a common chamber in said manifold connected to the gaseous medium supply.

In this case, the common chamber may be elongate and the gaseous medium is supplied to both ends of the chamber.

Preferably, each of said entraining channels includes a container having a chamber through which the gaseous medium flows and within which said substance is located. Preferably, the chamber is adapted to contain a granular or powder carrier for the substance and the container is removable from said channel for replacement when the substance therein is exhausted.

In a preferred embodiment, two of said manifolds are arranged with their flow paths in parallel.

In a further preferred embodiment, the apparatus includes at least one further channel to enable a predetermined portion of gaseous medium to be added to the flow in the outlet channels, downstream of said containers, to dilute by volume the amount of substance in the gaseous medium flowing to the outlets. In this case, two further channels may be provided each adapted to add a different predetermined portion to the gaseous medium.

Preferably, the entraining channels are all connected to further manifold devices in fluid communication with said outlets to ensure that the outflow at the outlets is substantially equal.

A preferred embodiment of the present invention will now be described by way of example with reference to the accompanying drawings, in which:-
Figure 1 shows a schematic view of gaseous fluid dispensing apparatus,
Figures 2a, 2b and 2c show sectional views through a container adapted to contain a substance to be dispensed.

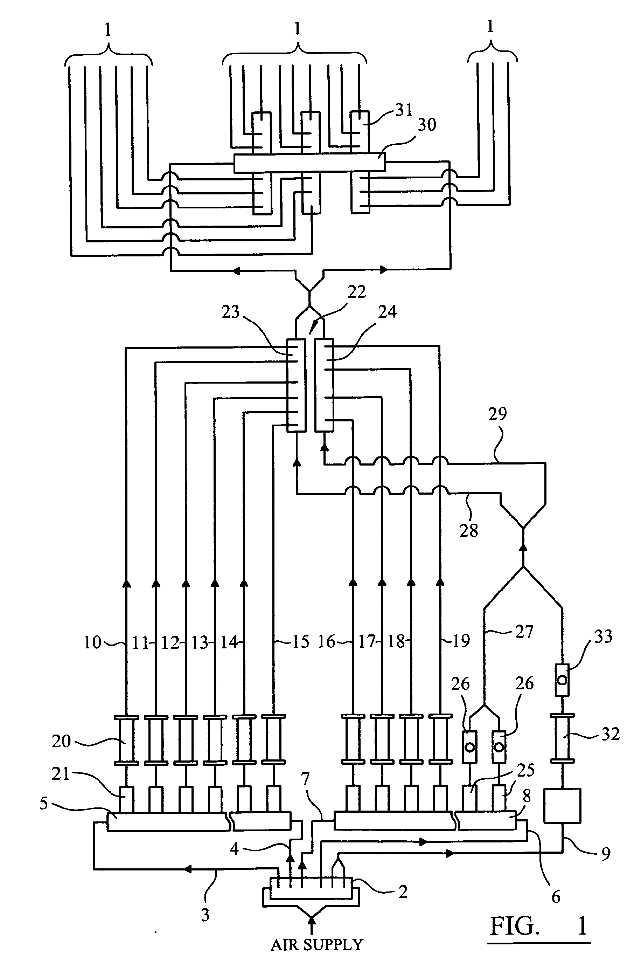

The gaseous fluid dispensing apparatus illustrated schematically in Figure 1 is intended to provide a flow of air to a plurality of outlets to which selected substances such as fragrances have been added. In this particular embodiment, the apparatus is intended for use in theatres and cinemas in which an outlet is provided adjacent to each seat and through which appropriate scents can be supplied to give an enhanced appreciation of the performance being viewed.

The apparatus is connected to a source of air and includes a first air dispersal manifold 2 having a plurality of outlets. As shown, the manifold has two outlets 3, 4 which lead to respective ends of a distribution manifold 5 having an elongate distribution chamber. Two further outlets, 6 and 7, are applied to the respective ends of a second distribution manifold 8. The object of supplying a to both ends of the manifold is to ensure as accurately as possible that all the outlet channels leading from the manifolds 5 and 8 get equal flow and pressure of air. A further outlet from the dispersal manifold 2 comprises a bypass channel 9.

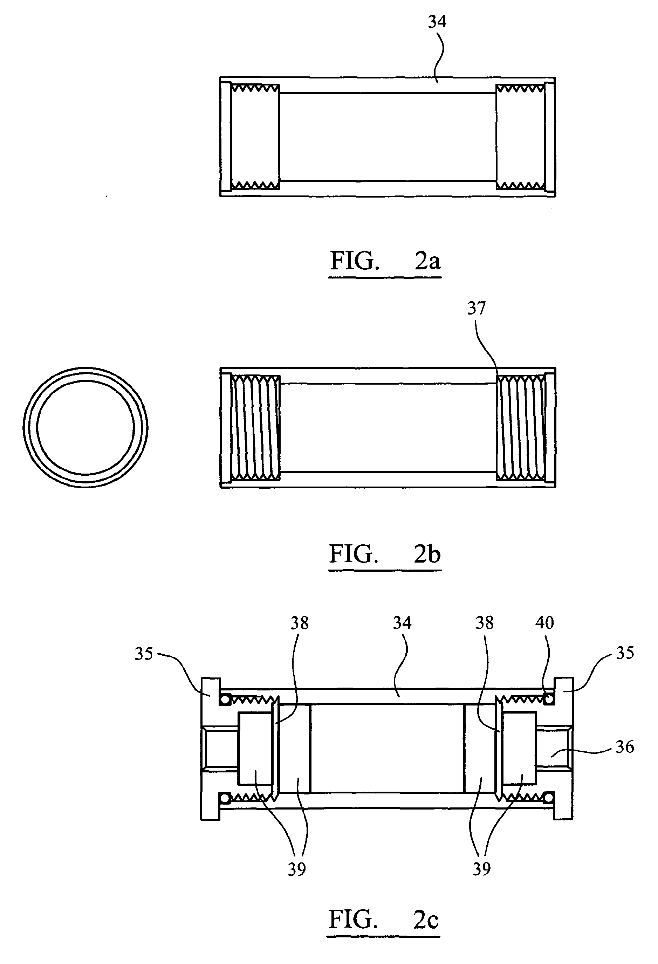

A plurality of output channels 10-19 are each connected in parallel to the respective distribution manifolds 5 and 8 through an associated solenoid shut off valve 21 mounted on the distribution manifolds to control the flow of air through the associated outlet channel. Each channel 10-19 includes in its flow path a scent cylinder 20, to be described in greater detail with reference to Figures 2a, 2b and 2c.

The output channels 10-19 are connected to a further manifold arrangement 22 which consists of two banks 23, 24 arranged in parallel, the outlet channels10-15 of the manifold 5 being connected to one bank 23 and the outlet channels 16-19 from the second manifold 8 being connected to the other bank 24.

The manifold 8 contains two further solenoid valves 25 which control the flow of air through two flow regulators 26. This allows a regulated flow of fresh air into a further channel 27 which is then split into two channels each of which leads to a respective one of the banks 23, 24 of the manifold 22. The flow regulators each allow a different percentage of air, such as 5% and 10%, to be sent to the manifold 22 to provide a required degree of dilution to the substances in the air passing through the outlet channels 10-19. Both flow regulators can be open to provide an increase in air flow of 15%.

From the manifold 22 the output air is applied to respective ends of a branch manifold 30 which includes a plurality of distribution ports 31 from each of which a plurality of the final outlets 1 extends to the final location. By means of splitting the air evenly and applying it to both ends of the manifold 30 and to ensuring that the distribution ports 31 each are of a similar size and have a similar number of outlets variation in the volume of the air flowing to each of the individual outlets 1 is kept as low as possible.

The bypass channel 9 is intended to provide a flow of air through the system to purge it of the air in the section from the manifold 22 to the outlets 1. The bypass channel 9 includes a bypass shut off valve 41 and a container 32 similar to the containers 20 in the outlet channels 10 which is incorporated in the flow past to ensure that the pressure drop through the bypass channel 9 is essentially the same as that of the outlet channels 10-19. Fine adjustment of the air flow through the bypass channel is carried out by means of a flow switch 33.

Referring now to Figures 2a, 2b and 2c, there is shown sectional views through a casing of a container 20 which is adapted to contain a carrier material such as granules or a powder coated or impregnated with a substance which is capable of being entrained by air passing through the container. The substance is typically a scent or fragrance of a volatile nature, but it is envisaged that other materials may be used, to dispense smoke for example. The container has a cylindrical main body 34 having an internal thread at each end into which a cap 35 containing a connector 36 to which the pipework of the associated outlet channel 10-19 can be connected. The thread in the container body ends in a shoulder 37 which supports a flat disc 38 formed of a mesh material which is thus clamped between the container housing 34 and the inner end of the cap 35. Filtration material is located on either side of the disc 38. The interior of the container is filled with a granular or powder material impregnated with a scent or fragrance which is entrained by air passing through the container. Filtration material is located on either side of the disc 38 to prevent granular or powder material being drawn into the channels 10-19. An O ring seal 40 is provided between the cap 35 and the housing 34 to give an airtight seal.

By means of the threaded caps 35, the containers 20 can be easily and readily replaced when their substances are exhausted.

In operation, when it is desired to provide a certain fragrance at the outlets 1, the appropriate solenoid valve 21 is opened and air passes from the chamber in the manifold, the open solenoid valve 21, through the associated channel 10-19 and container 20 from where the particular scent is passed through the manifold systems to the outlets 1. It is envisaged that more than one solenoid valve 21 may be open at the same time to provide a desired mix of fragrances but in general it is anticipated that only one valve will be open at a time. The effect of the shut-off valves is such that when they are closed it is impossible for any significant amount of the gas in the channels 10-19 can be entrained by air passing through the manifold 22. In addition, by operation of the overflow channel 9 when all the solenoid valves are closed, existing fragrance in the outlets can be purged before the next fragrance is released.

Although described with reference to the provision of suitable smells and fragrances for use in the theatre and cinema, it will be appreciated that the apparatus is suitable for many other applications where it is desired to provide a particular ambiance at a particular location. For example, the apparatus could be used to supply fragrance, disinfectants or even drugs such as provided by nebulisers, to bedridden people at home or in hospital.

## Claims

1. Apparatus for dispensing a gaseous medium to a plurality of outlets, the apparatus including means to add selectively a substance or substances to said gaseous medium, the apparatus including a first manifold to which the gaseous medium is supplied the manifold having connected thereto a plurality of entraining channels each containing a substance to be entrained by the gaseous medium passing therethrough, each entraining channel having a closure valve selectively operable so that the gaseous medium is able to pass through a selected channel or channels only.

2. Apparatus according to claim 1 including a bypass channel to enable pure gaseous medium to be supplied downstream of the containers to purge the channels of entrained substances.

3. Apparatus according to claim 1 wherein the closure valves are mounted on the manifold so as to be in fluid communication with a common chamber in said manifold adapted to be connected to the gaseous medium supply.

4. Apparatus according to claim 3 wherein the common chamber is elongate and the gaseous medium is supplied to both ends of the chamber.

5. Apparatus according to any one of the preceding claims wherein each of said entraining channels includes a container having a chamber through which the gaseous medium flows and within which said substance is located.

6. Apparatus according to claim 5 wherein the chamber is adapted to contain a granular or powder carrier for the substance and the container is removable from said channel for replacement when the substance therein is exhausted.

7. Apparatus according to any one of the preceding claims wherein two of said manifolds are arranged with their flow paths in parallel.

8. Apparatus according to any one of the preceding claims including at least one further channel to enable a predetermined portion of gaseous medium to be added to the flow in the outlet channels, downstream of said container to dilute by volume the amount of substance in the gaseous medium flowing to the outlets.

9. Apparatus according to claim 8 wherein two further channels are provided each adapted to add a different predetermined portion to the gaseous medium.

10. Apparatus according to any one of the preceding claims wherein the entraining channels are all connected to further manifold devices in fluid communication with said outlet to ensure that the outflow at the outlets is substantially equal.

11. Apparatus according to claim 10 when dependent on claim 2, wherein the bypass channel is connected to the first manifold to which the outlet channels are connected downstream of the containers.

12. Apparatus according to any one of the preceding claims wherein the gaseous medium is air.
